# EUROPEAN PATENT SPECIFICATION

(11) **EP 3 172 374 B1**
(45) Date of publication and mention of the grant of the patent: **17.04.2019**
(21) Application number: 16703475.0
(22) Date of filing: 02.02.2016
(51) Int. Cl.: D06F 58/20, D06F 58/26, D06F 58/10

(54) **GARMENT CARE DEVICE FOR DRYING AND SANITIZING GARMENTS AND METHOD OF TREATING A GARMENT IN A GARMENT CARE DEVICE**
KLEIDERPFLEGEVORRICHTUNG ZUM TROCKNEN UND DESINFIZIEREN VON KLEIDERN UND VERFAHREN ZUR BEHANDLUNG VON KLEIDERN
DISPOSITIF D'ENTRETIEN DE VÊTEMENT SERVANT À SÉCHER ET DÉSINFECTER DES VÊTEMENTS ET PROCÉDÉ DE TRAITEMENT DES VÊTEMENTS

(30) Priority: 03.02.2015 EP 15153528
(43) Date of publication of application: 31.05.2017
(73) Proprietor: Koninklijke Philips N.V., 5656 AE Eindhoven (NL)
(72) Inventor: ZHAO, Lihong, 5656 AE Eindhoven Netherlands (NL); PNG, Luck, Wee, 5656 AE Eindhoven NL (NL); JIANG, Yong, 5656 AE Eindhoven Netherlads (NL); POPESCU, Crisan, 5656 AE Eindhoven Netherlands (NL)
(74) Representative: Uittenbroek, Arie Leendert
(86) International application number: PCT/EP2016/052196
(87) International publication number: WO 2016/124606

(56) References cited:
- EP-A1- 2 113 605
- EP-A1- 2 759 633
- DE-A1-102009 026 712
- JP-A- 2005 253 786
- KR-A- 20060 018 432

## Description

### FIELD OF THE INVENTION

The invention relates to a garment care device for drying and sanitizing garments, and also relates to a corresponding method for drying and sanitizing garments.

The invention may be used, for example, in the field of garment care.

### BACKGROUND OF THE INVENTION

Conventional washing is not able to remove all bacteria on garment, and a mouldy odour develops if the garments are left wet for a long time. This issue can be partially solved by using garment dryers, such as tumble dryers. However, the high heats generated in the drum of the dryer often cause garments to shrink, and severe crease at the edges and fray of garments. So despite the growth in popularity of tumble dryers in recent years, people still prefer sun drying, for example in Asian countries. Sunshine drying of garments is popular as natural ultraviolet radiation has a sanitizing effect on garments, can remove mouldy odour and replacing it with a pleasant scent to indicate thorough sanitization. This pleasant scent is sometimes called "smell of the sun" by people. Moreover, sun drying causes much less damage to garments, and natural ultraviolet sterilization is perceived to be significantly better than hot air and tumbling action provided by known dryers. However, due to increasing levels of air pollution in most urban areas, outdoor sun-dried garments get contaminated. Moreover, prolonged rainy seasons can also deprive people of the sun-dry benefits. Therefore, sun-dried technology is highly desired at indoor conditions without the interference of outdoor ambient conditions.

Some known solutions describe technology to treat garments in an indoor environment while giving this pleasant scent referred to as "smell of the sun". Those known solutions use the combination of ultraviolet radiation and ozone.

However, ozone is known to be potentially harmful for people as well as for the ecosystem, so alternative solutions are desired. A sterilization dryer and a method of treating a garment with said sterilization dryer according to the preamble of claims 1, 12, 13 and 14 is known from KR20060018432A.

### OBJECT AND SUMMARY OF THE INVENTION

It is an object of the invention to propose an improved garment care device that avoids or mitigates above-mentioned problems.

The invention is defined by the independent claims. The dependent claims define advantageous embodiments.

To this end, the garment care device according to an aspect of the invention comprises:
- a chamber to receive at least one garment,
- a heating system for heating the at least one garment,
- a lighting system for emitting inside the chamber, a radiation having wavelength in the range from 280 nm to 500 nm,
- a metal oxide photocatalyst element disposed inside the chamber such that the metal oxide photocatalyst element can receive the radiation, wherein the chamber (C) comprises receiving means (H, T) to receive said at least one garment (G) such that said at least one garment (G) remains spaced from said metal oxide photocatalyst element (MO), characterized in that said metal oxide photocatalyst element (MO) takes the form of at least one layer in contact with a wall of said chamber (C), said wall being taken among the set defined by bottom wall, lateral wall, rear wall, front wall, and top wall.

A garment care device according to another aspect of the invention comprises:
- a chamber (C) to receive at least one garment (G),
- a heating system (HS) for heating said at least one garment (G),
- a lighting system (LS) for emitting inside said chamber (C), a radiation having wavelength in the range from 280 nm to 500 nm,
- a metal oxide photocatalyst element (MO) disposed inside said chamber (C) such that the metal oxide photocatalyst element (MO) can receive said radiation,
   wherein the chamber (C) comprises receiving means (H, T) to receive said at least one garment (G) such that said at least one garment (G) remains spaced from said metal oxide photocatalyst element (MO), characterized in that:
   - said lighting system (LS) and said metal oxide photocatalyst element (MO) are disposed adjacent to a same given wall of said chamber (C), said same given wall being
   - taken among the set defined by bottom wall, lateral wall, rear wall, front wall, and top wall, and
   - said metal oxide photocatalyst element (MO) takes the form of a radiation reflector (LR) disposed between said lighting system (LS) and said same given wall.

A garment care device according to yet another aspect of the invention comprises:
- a chamber (C) to receive at least one garment (G),
- a heating system (HS) for heating said at least one garment (G),
- a lighting system (LS) for emitting inside said chamber (C), a radiation having wavelength in the range from 280 nm to 500 nm,
- a metal oxide photocatalyst element (MO) disposed inside said chamber (C) such that the metal oxide photocatalyst element (MO) can receive said radiation, wherein the chamber (C) comprises receiving means (H, T) to receive said at least one garment (G) such that said at least one garment (G) remains spaced from said metal oxide photocatalyst element (MO), characterized in that:
   - said lighting system (LS) and said metal oxide photocatalyst element (MO) are disposed adjacent to a same given wall of said chamber (C), said same given wall being taken among the set defined by bottom wall, lateral wall, rear wall, front wall, and top wall, and
   - said metal oxide photocatalyst element (MO) takes the form of a perforated cover (PC), said lighting system (LS) being disposed between said metal oxide photocatalyst element (MO) and said same given wall.

According to the invention, there is also provided a method of treating a garment in a garment care device according to claim 12.

The garment care device according to the invention allows the combination of drying and sanitizing of garments. Drying of garment is done via heating, and sanitizing of garment is done via the radiation generated by the lighting system. Also, by irradiating the metal oxide photocatalyst element with light radiation having wavelength in the range [280; 500] nm in the chamber, the pleasant smell referred to as "smell of the sun" is generated and imparted to the garment in the chamber, and the garment keep this particular smell even after being taken out of the chamber. Moreover, this characteristic smell is generated without the need of using ozone, which makes this solution sustainable in terms of protecting people and environment.

Detailed explanations and other aspects of the invention will be given below.

### BRIEF DESCRIPTION OF THE DRAWINGS

Particular aspects of the invention will now be explained with reference to the embodiments described hereinafter and considered in connection with the accompanying drawings, in which identical parts or sub-steps are designated in the same manner :
Fig.1 depicts a first simplified embodiment example of a garment care device according to the invention,
Fig.2 depicts a second simplified embodiment example of a garment care device according to the invention,
Fig.3 depicts a garment care device according to the invention,
Fig.4 illustrates the reaction happening when a metal oxide photocatalyst element is irradiated with a radiation having wavelength in the range [280; 500] nm,

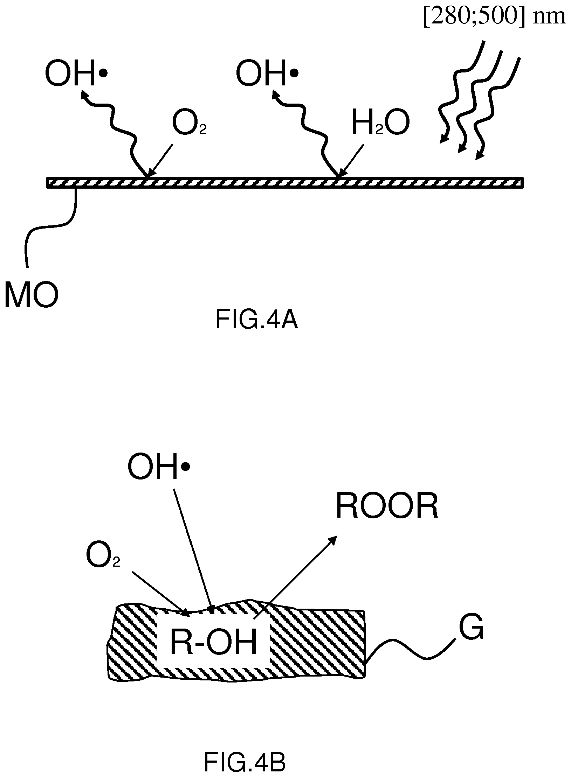

Fig.4 illustrates the reaction happening between hydroxide and garment,

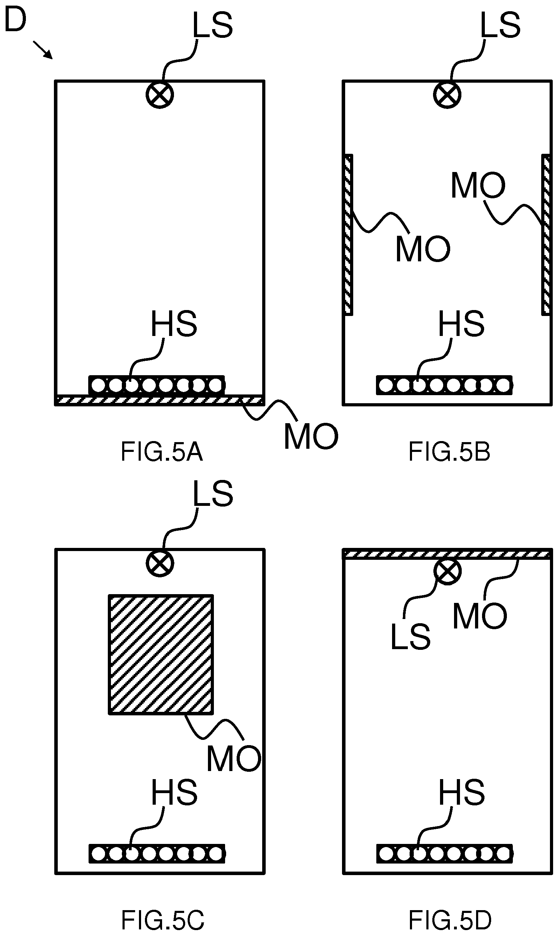

Fig.5A to Fig.5H depict various preferred embodiments of a garment care device according to the invention,

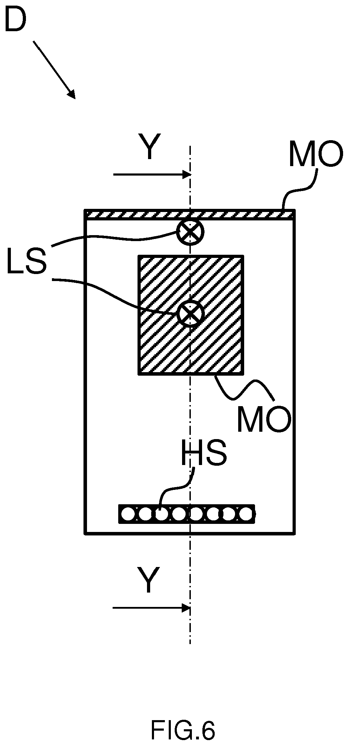

Fig.6 depicts a preferred embodiment of a garment care device according to the invention,

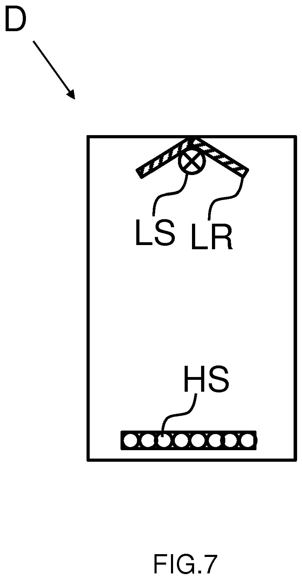

Fig.7 depicts an embodiment of a garment care device according to the invention comprising a radiation reflector,

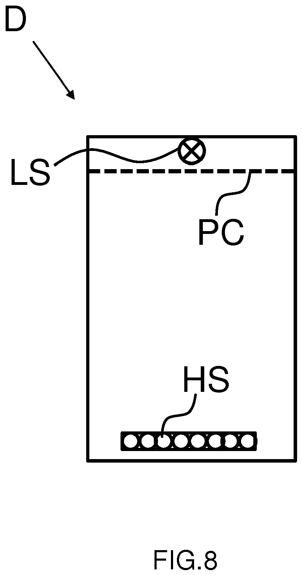

Fig.8 depicts an embodiment of a garment care device according to the invention comprising a perforated cover,

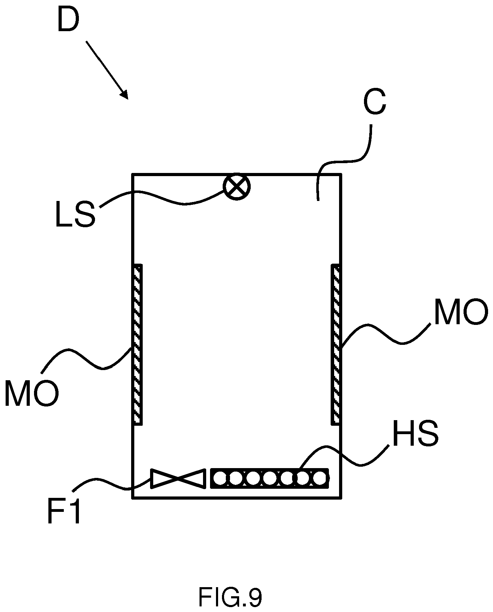

Fig.9 depicts an embodiment of a garment care device according to the invention comprising a first fan,

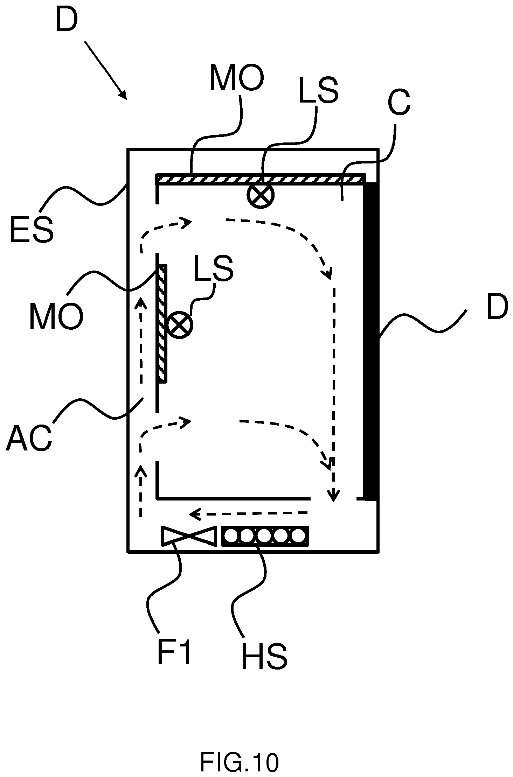

Fig.10 depicts a first embodiment of a garment care device according to the invention comprising a first fan and an external shell,

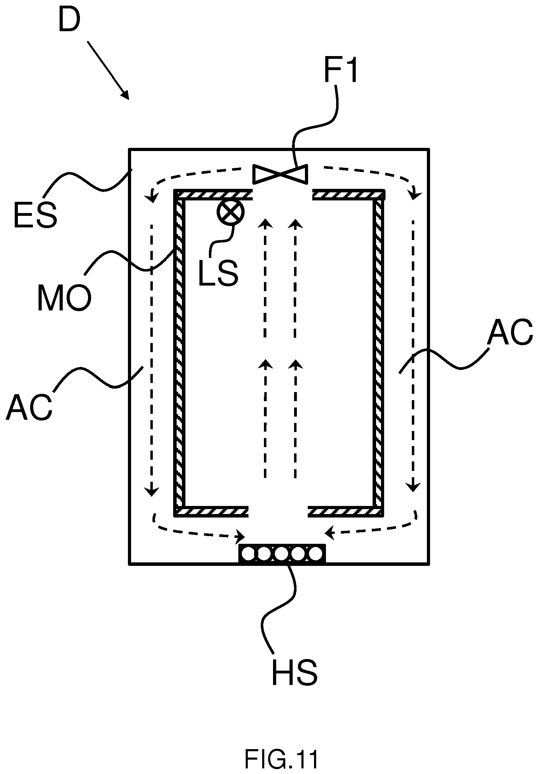

Fig.11 depicts a second embodiment of a garment care device according to the invention comprising a first fan and an external shell,

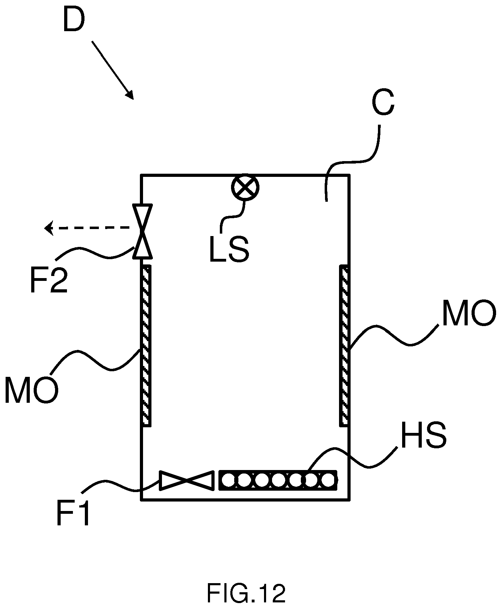

Fig.12 depicts a first embodiment of a garment care device according to the invention comprising a first fan and a second fan,

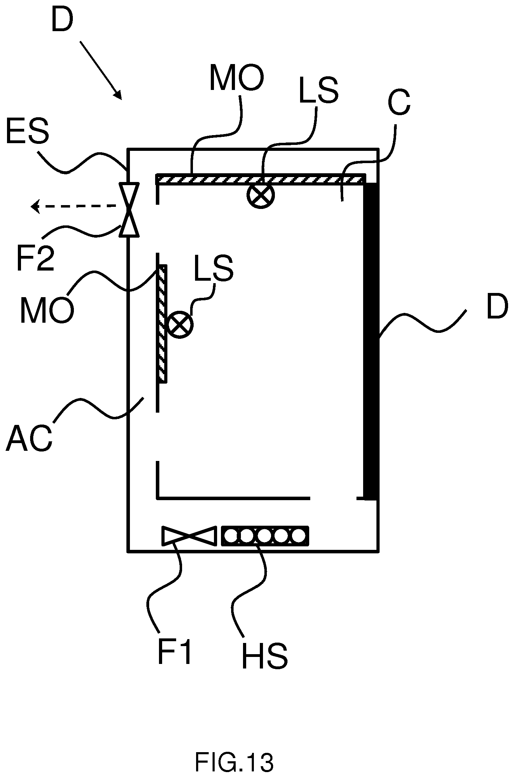

Fig.13 depicts a second embodiment of a garment care device according to the invention comprising a first fan and a second fan.

### DETAILED DESCRIPTION OF THE INVENTION

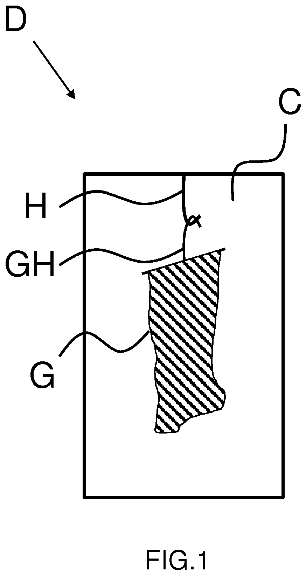

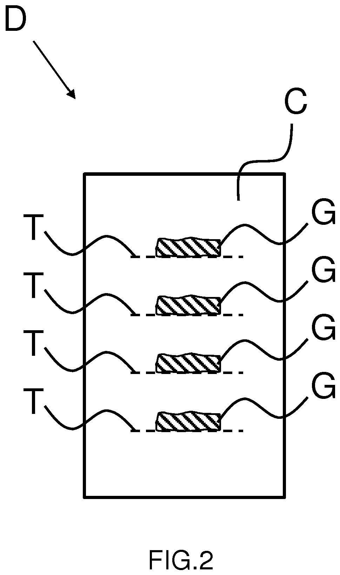

Fig.1 and Fig.2 depict simplified embodiment examples of embodiments of a garment care device (D) according to the invention for sanitizing/drying garments. The garment care device (D) comprises a chamber (C) to receive at least one garment (G). For example, the chamber (C) forms a closed cabinet, such as with a parallelepiped shape with bottom wall, lateral wall, rear wall, front wall, and top wall. For example, a door (not shown) allows accessing the inside of the chamber C by a user to dispose garments therein and take our garments after the sanitizing/drying is done.

In a first example depicted in Fig.1, there is depicted a garment care device (D) according to the invention comprising a chamber C where at least one garment G is hanged vertically for conducting the sanitizing/drying.

In a second example depicted in Fig.2, there is depicted a garment care device (D) according to the invention comprising a chamber C where at least one garment G is disposed (folded) on a flat support for conducting the sanitizing/drying.
In the following, for sake of clarity, the garment(s) disposed inside the chamber C will not be represented on the various figures illustrating the garment care device D according to the invention.

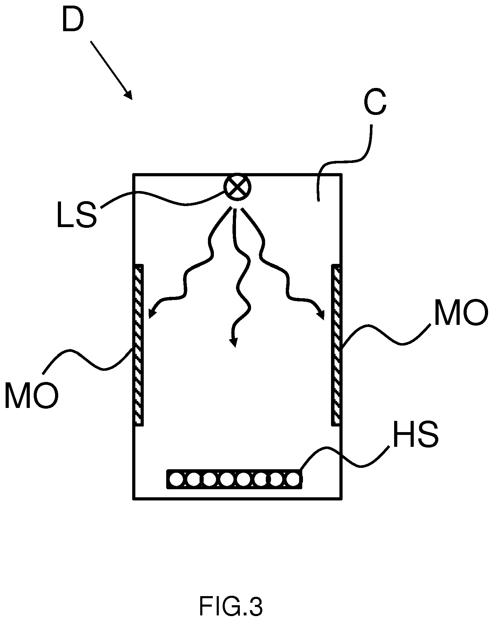

Fig.3 depicts a garment care device (D) according to the invention.

The garment care device (D) comprises a chamber (C) to receive at least one garment (G), as described previously along with Fig.1 and Fig.2.

The garment care device (D) also comprises a heating system (HS) for heating the at least one garment (G). For example, as illustrated, the heating system (HS) is disposed on the bottom wall of the chamber C. Alternatively, the heating system (HS) can also be disposed at other locations of the chamber C, as long as it can heat the garment inside the chamber C. Preferably, the heating system (HS) is adapted to heat garment in the chamber C with a temperature in the range [40,70] °C. For example, the heating system (HS) is a resistive element supplied by an electric current and generating heat towards the garment via convection. Alternatively, the heating system (HS) is a resistive element supplied by an electric current and generating heat towards the garment via (infrared) radiation. Alternatively, the heating system (HS) is a resistive element supplied by an electric current and generating heat towards the garment via convection and (infrared) radiation. For example, the sanitizing/drying duration cycle is in the range [15;60] mn.

The garment care device (D) also comprises a lighting system (LS) for emitting inside the chamber C, a radiation having wavelenght in the range of [280; 500] nm. The radiation is illustrated by three wavy arrows. In this embodiment, the lighting system (LS) is disposed on the top wall of the chamber C. However, other locations in the chamber C for the lighting system (LS) are also possible, as it will be described in the following.

The garment care device (D) also comprises a metal oxide photocatalyst element (MO) disposed inside the chamber (C) such that metal oxide photocatalyst element (MO) can receive the radiation emitted by the lighting system (LS). In this illustration, the metal oxide photocatalyst element (MO) is disposed on the lateral walls of the chamber C. However, other locations in the chamber C for the metal oxide photocatalyst element (MO) are also possible, as it will be described in the following,.

The chamber (C) comprises receiving means to receive the at least one garment (G) such that the at least one garment (G) remains spaced from the metal oxide photocatalyst element (MO).

For example, as depicted in Fig.1, the receiving means comprise a hook H fixed in the top wall of the chamber C so that the at least one garment G is hanged/suspended in the chamber C (directly, or via using a detachably garment hanger GH).

For example, as depicted in Fig.2, the receiving means comprise (slideable) trays T having (air permeable) horizontal (or alternatively inclined) structure to support the at least one garment G. In Fig.2, four trays T are illustrated. After the door (not shown) of the chamber C is opened, trays T are pulled out from the chamber C by a user, in order for the user to dispose garments thereon. Then, the trays T are pulled back inside the chamber C by the user, and the door of the chamber C is closed by the user.

In Fig.3 to Fig.13, the receiving means will not be represented for sake of clarity.

When the metal oxide photocatalyst element (MO) is irradiated by radiation having wavelenght in the range of [280; 500] nm, as illustrated by Fig.4A, electron and hole pairs are generated at the surface of the metal oxide photocatalyst element (MO). Electron and hole pairs further react with O₂ and H₂O contained in the air of the chamber C to form free radicals of hydroxide OH*. Then, as illustrated in Fig.4B, if the garment G contains some natural fibers of the form R-OH, where R represents any organic radical, OH• extracts H from R-OH to form organic free radical R•. For example, natural fibers may correspond to cotton, linen, wool. For example, if garment fibers contain cotton comprising cellulose, R corresponds to CH₂. The free radical R• then reacts with O₂ in the air to form organic peroxide ROOR which is one contributor (among others) to generate the so-called "smell of the sun.

For example, if the lighting system (LS) is adapted to emit an ultraviolet radiation in the UVB range [280; 315] nm, and if the metal oxide photocatalyst element (MO) corresponds to titanium oxide having chemical formula TiO₂, the reaction occurring in the chamber C can be summarized as follows:

TiO₂ + UVB → e⁻ (electron) + h⁺ (hole)

h⁺ (hole) + H₂O → OH^{•} + H+

e⁻ (electron) + O₂ → O₂^{•-}

O2^{•-} + 2H₂O → 2 OH^{•} + 2OH⁻ + O₂

Preferably, the metal oxide photocatalyst element (MO) takes the form of at least one layer disposed on a wall of the chamber (C), the wall being taken among the set defined by bottom wall, lateral wall, rear wall, front wall, and top wall. Statement "on a wall" refers either to configurations where the at least one layer is contact with the wall, or where the at least one layer is at proximity of the wall. This implies that any combination of those different positions for the metal oxide photocatalyst element (MO) is also possible. The layer of metal oxide photocatalyst element (MO) does not necessarily covers all the surface of the corresponding wall. However, it is preferred that the surface of the plate of metal oxide photocatalyst element (MO) covers at least 1% of the surface of the wall it is disposed thereon.
The layer of metal oxide photocatalyst element (MO) either corresponds to a plate coated with metal oxide photocatalyst element (MO) and fixed on a wall of the chamber, or to a coating of metal oxide photocatalyst element (MO) directly made on a wall of the chamber C. For example, the coating of metal oxide photocatalyst element (MO) has a thickness of a few nanometers. The layer of metal oxide photocatalyst element (MO) either forms a unique surface, or a plurality of separate surfaces disposed at different locations on wall(s) of the chamber C.

Below are non-limitative preferable examples of embodiments showing the metal oxide photocatalyst element (MO) disposed at different locations in the chamber C (the lighting system S is disposed on the top wall of the chamber C, but could be disposed at other positions as it will be described in the following):
- Fig.5A depicts a front view of a garment care device (D) according to the invention where the metal oxide photocatalyst element (MO) is disposed on the bottom wall of the chamber (C).
- Fig.5B depicts a front view of a garment care device (D) according to the invention where the metal oxide photocatalyst element (MO) is disposed on lateral walls of the chamber (C). It is noted that disposing the metal oxide photocatalyst element (MO) on only one of the lateral wall is also possible (not shown).
- Fig.5C depicts a front view of a garment care device (D) according to the invention where the metal oxide photocatalyst element (MO) is disposed on the rear wall of the chamber (C).
- Fig.5D depicts a front view of a garment care device (D) according to the invention where the metal oxide photocatalyst element (MO) is disposed on the top wall of the chamber (C).

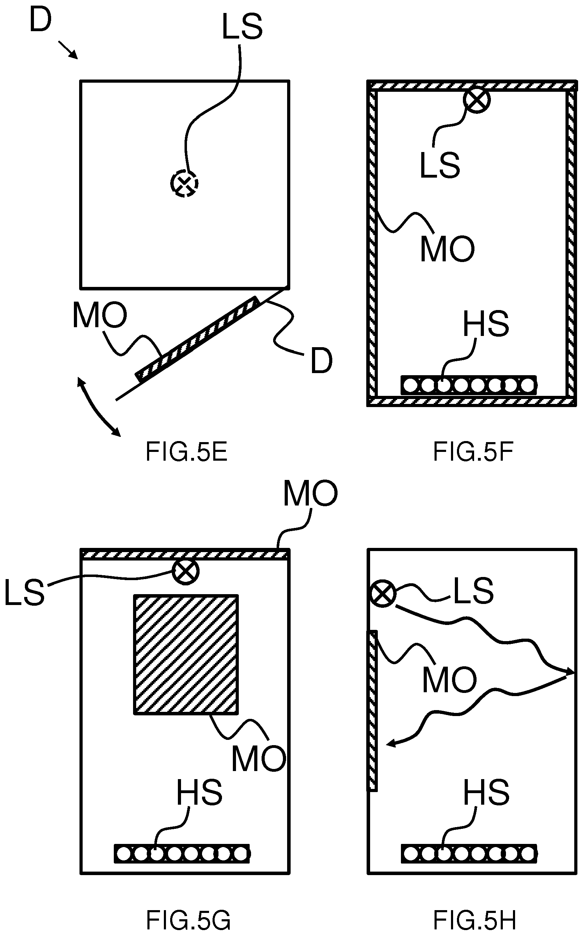

- Fig.5E depicts a top view of a garment care device (D) according to the invention where the metal oxide photocatalyst element (MO) is disposed on the front wall of the chamber (C). The front wall corresponds to a door D that can be opened by a user to access the inside part of the chamber C.
- Fig.5F depicts a front view of a garment care device (D) according to the invention where the metal oxide photocatalyst element (MO) is disposed all around the walls of the chamber (C).
- Fig.5G depicts a front view of a garment care device (D) according to the invention where the metal oxide photocatalyst element (MO) is disposed on the top wall of the chamber (C) and on the rear wall of the chamber (C).

Preferably, the lighting system (LS) is disposed adjacent to a given wall of the chamber (C) taken among the set defined by bottom wall, lateral wall, rear wall, front wall, and top wall. This also means that the lighting system (LS) is not necessarily disposed on the same wall as the wall on which the metal oxide photocatalyst element (MO) is disposed thereon. Indeed, the radiation can irradiate the metal oxide photocatalyst element (MO) at least partly by direct radiation (as illustrated in Fig.5A to Fig.5G), and/or at least partly by indirect radiation (via reflection of the radiation on the various internal wall(s) of the chamber C, as illustrated in Fig.5H).

Preferably, the lighting system (LS) and the metal oxide photocatalyst element (MO) are disposed adjacent to a same given wall of the chamber (C) taken among the set defined by bottom wall, lateral wall, rear wall, front wall, and top wall.

Having the lighting system (LS) adjacent to a wall on which the metal oxide photocatalyst element (MO) is also disposed ensures a better irradiation of the metal oxide photocatalyst element (MO) to the radiation. As a result, the generation of free radicals OH• is improved. This characteristic is illustrated for example on Fig.5D, Fig.5F, and Fig.5G.

Another preferable example is depicted by Fig.6 which illustrates a garment care device (D) according to the invention where the metal oxide photocatalyst element (MO) is disposed for one part on the top wall of the chamber (C) and for a second part on the rear wall of the chamber (C). The lighting system (LS) is for one part disposed adjacent to the metal oxide photocatalyst element (MO) disposed on the top wall, and for a second part disposed adjacent to the metal oxide photocatalyst element (MO) disposed on the rear wall of the chamber (C).

Preferably, as depicted in Fig.7, when the lighting system (LS) and the metal oxide photocatalyst element (MO) are disposed adjacent to a same given wall of the chamber (C), the metal oxide photocatalyst element (MO) takes the form of a radiation reflector (LR) disposed between the lighting system (LS) and the same given wall on which the metal oxide photocatalyst element (MO) is disposed. The radiation reflector (LR) ensures that a larger surface of the metal oxide photocatalyst element (MO) is directly irradiated by the radiation. As a result, the generation of free radicals OH• is further improved. The radiation reflector (LR) also allows to direct the radiation towards the garments in order to improve the sanitizing effect. For example, as illustrated, the radiation reflector (LR) forms a concave angle.

Preferably, as depicted in Fig.8, when the lighting system (LS) and the metal oxide photocatalyst element (MO) are disposed adjacent to a same given wall of the chamber (C), the metal oxide photocatalyst element (MO) takes the form of a perforated cover (PC). The lighting system (LS) is disposed between the metal oxide photocatalyst element (MO) and the wall the metal oxide photocatalyst element (MO) is facing. The plurality of openings formed in the perforated cover (PC) contributes to a more even reaction between the radiation and the metal oxide photocatalyst element (MO).

Preferably, the metal oxide photocatalyst element (MO) is chosen among the set of metal oxides defined by titanium dioxide having chemical formula TiO₂, iron oxide having chemical formula FeO, iron oxide having chemical formula Fe₂O₃, and iron oxide having chemical formula Fe₃O₄. It is noted that any other metal oxide photocatalysts could be used as long as they can form free radicals OH• when irradiated by the radiation having wavelength in the range [280; 500] nm.

Preferably, the lighting system (LS) is adapted to emit radiation with wavelength in the Ultraviolet A (UVA) range [315; 400] nm, which is advantageous considering the current relatively cheaper price of the corresponding lighting system.

Preferably, the lighting system (LS) is adapted to emit radiation with wavelength in the Ultraviolet B (UVB) range [280; 315] nm.

Preferably, the lighting system (LS) comprises a single light bulb, or a single light tube.

Preferably, the lighting system (LS) comprises a plurality of lighting units, such as a plurality of light bulbs, and/or a plurality of lighting tubes.

Preferably, the lighting system (LS) is adapted to emit radiation with a lighting energy of at least 2 kilo Joules/m2, for example 4 kilo Joules/m2. This ensures that the metal oxide photocatalyst element (MO) and the surface of garment receive sufficient amount of energy.

Preferably, the garment care device (D) according to the invention further comprises a first fan (F1) to circulate air inside the chamber (C). An example of this preferred embodiment is depicted in Fig.9. This embodiment is based on the embodiment depicted in Fig.3, with an additional first fan F1 disposed on the bottom wall of the chamber C. The first fan F1 allows blowing air inside the chamber, so that the heated air is more evenly distributed and circulated inside the chamber C. This contributes to a faster and more efficient drying of the garments. Alternatively, the first fan F1 can be disposed at any other positions in the chamber C, such as near the top wall or the lateral walls of the chamber C.

Preferably, the garment care device (D) according to the invention further comprises an external shell (ES) enclosing the chamber (C). The heating system (HS) and the first fan (F1) are disposed in an air channel (AC) formed between the external shell (ES) and the chamber (C), to circulate a flow of hot air in the chamber (C). This circulation of hot air inside the chamber C contributes to a faster and more efficient drying of the garments,.

A first example of an embodiment using the first fan F1 is depicted in Fig.10. This embodiment is based on the embodiment depicted in Fig.6 with a representation along axis Y-Y, and comprising an additional external shell (ES). The flow of hot air is illustrated by the various arrows in dotted line style. The flow of hot air is circulated in the air channel (AC) which extends below the bottom wall and behind the rear wall of the chamber C. The first fan F1 and the heating system (HS) are for example disposed in the air channel AC below the bottom wall of the chamber C.

A second example of an embodiment using the first fan F1 is depicted in Fig.11. This embodiment is based on the embodiment depicted in Fig.5F, and comprising an additional external shell (ES). The flow of hot air is illustrated by the various arrows in dotted line style. The flow of hot air is circulated in the air channel (AC) which extends below the bottom wall and behind the two lateral walls of the chamber C. The first fan F1 is disposed in the air channel AC above the top wall of the chamber C. The heating system (HS) is disposed in the air channel AC below the bottom wall of the chamber C. It is noted that the position of the first fan F1 and the position of the heating system (HS) could be interchanged to achieve a similar result.

Preferably, the first fan (F1) is adapted to circulate a flow of hot air in the chamber (C) with a speed in the range [0.01; 1] m/s. For example, the speed of the air flow can be adjusted by varying the rotation speed of the first fan (F1). The higher air is circulated in the chamber the faster the drying of garment.

Preferably, the garment care device (D) further comprises a second fan (F2) to draw air out of the chamber (C). By drawing air out of the chamber (C), the humidity of air inside the chamber C can be removed, in order to increase the drying of garments. For example, the second fan (F2) is activated when the humidity level in the chamber C exceeds a given threshold.

A first example of an embodiment using the second fan F2 is depicted in Fig.12. This embodiment is based on the embodiment depicted in Fig.9. The flow of air which is drawn from the chamber C is illustrated by the arrow in dotted line style. The second fan F2 is preferably disposed in the upper part of the chamber C, for example along a lateral wall of the chamber C (as illustrated), or the rear wall of the chamber C (not shown).

A second example of an embodiment using the second fan F2 is depicted in Fig.13. This embodiment is based on the embodiment depicted in Fig. 10. The flow of air drawn from the chamber C is illustrated by the arrow in dotted line style. The second fan F2 is preferably disposed in the upper part of the chamber C, for example along a lateral wall of the chamber C (not shown), or the rear wall of the chamber C (as illustrated).

According to the invention, there is also provided a method (not illustrated by a flow chart) of treating a garment in a garment care device (D) as described above and comprising:
- a chamber (C) to receive at least one garment (G),
- a heating system (HS) for heating the at least one garment (G),
- a lighting system (LS) for emitting inside the chamber (C), a radiation having wavelength in the range [280; 500] nm,
- a metal oxide photocatalyst element (MO) disposed inside the chamber (C) such that the metal oxide photocatalyst element (MO) can receive the radiation.

The method comprises the step of disposing the at least one garment (G) within the chamber (C) such that the at least one garment (G) remains spaced from the metal oxide photocatalyst element (MO).

The above embodiments as described are only illustrative, and not intended to limit the technique approaches of the present invention. Although the present invention is described in details referring to the preferable embodiments, those skilled in the art will understand that the technique approaches of the present invention can be modified or equally displaced without departing from the scope of the claims of the present invention. In the claims, the word "comprising" does not exclude other elements or steps, and the indefinite article "a" or "an" does not exclude a plurality. Any reference signs in the claims should not be construed as limiting the scope.

## Claims

1. A garment care device (D) comprising:
- a chamber (C) to receive at least one garment (G),
- a heating system (HS) for heating said at least one garment (G),
- a lighting system (LS) for emitting inside said chamber (C), a radiation having wavelength in the range from 280 nm to 500 nm,
- a metal oxide photocatalyst element (MO) disposed inside said chamber (C) such that the metal oxide photocatalyst element (MO) can receive said radiation,
wherein the chamber (C) comprises receiving means (H, T) to receive said at least one garment (G) such that said at least one garment (G) remains spaced from said metal oxide photocatalyst element (MO), **characterized in that** said metal oxide photocatalyst element (MO) takes the form of at least one layer in contact with a wall of said chamber (C), said wall being taken among the set defined by bottom wall, lateral wall, rear wall, front wall, and top wall.

2. A garment care device (D) as claimed in claim 1, wherein said lighting system (LS) is disposed adjacent to a given wall of said chamber (C), said given wall being taken among the set defined by bottom wall, lateral wall, rear wall, front wall, and top wall.

3. A garment care device (D) as claimed in anyone of the preceding claims, wherein said metal oxide photocatalyst element (MO) is chosen among the set of metal oxides defined by titanium dioxide having chemical formula TiO₂, iron oxide having chemical formula FeO, iron oxide having chemical formula Fe₂O₃, and iron oxide having chemical formula Fe₃O₄.

4. A garment care device (D) as claimed in anyone of the preceding claims, further comprising a first fan (F1) to circulate air inside said chamber (C).

5. A garment care device (D) as claimed in claim 4, further comprising an external shell (ES) enclosing said chamber (C), said heating system (HS) and said first fan (F1) being disposed in an air channel (AC) formed between said external shell (ES) and said chamber (C), to circulate a flow of hot air in said chamber (C).

6. A garment care device (D) as claimed in claim 5, wherein said first fan (F1) is adapted to circulate a flow of hot air in said chamber (C) in the range from 0.01 m/s to 2 m/s.

7. A garment care device (D) as claimed in claim 4, further comprising a second fan (F2) to draw air out of said chamber (C).

8. A garment care device (D) as claimed in anyone of claims 1 to 7, wherein said lighting system (LS) is adapted to emit said radiation with wavelength in the range from 315 nm to 400 nm.

9. A garment care device (D) as claimed in anyone of claims 1 to 7, wherein said lighting system (LS) is adapted to emit said radiation with wavelength in the range from 280 nm to 315 nm.

10. A garment care device (D) as claimed in anyone of claims 1 to 7, wherein said lighting system (LS) comprises a plurality of lighting units.

11. A garment care device (D) as claimed in claim 8, 9 or 10, wherein said lighting system (LS) is adapted to emit said radiation with a lighting energy of at least 2 kilo Joules/m2.

12. A method of treating a garment in a garment care device (D) comprising:
- a chamber (C) to receive at least one garment (G),
- a heating system (HS) for heating said at least one garment (G),
- a lighting system (LS) for emitting inside said chamber (C), a radiation having wavelength in the range from 280 nm to 500 nm,
- a metal oxide photocatalyst element (MO) disposed inside said chamber (C) such that the metal oxide photocatalyst element (MO) can receive said radiation,
the method comprising the step of:
- disposing said at least one garment (G) within the chamber (C) such that the at least one garment (G) remains spaced from the metal oxide photocatalyst element (MO), **characterized in that** said metal oxide photocatalyst element (MO) takes the form of at least one layer in contact with a wall of said chamber (C), said wall being taken among the set defined by bottom wall, lateral wall, rear wall, front wall, and top wall.

13. A garment care device (D) comprising:
- a chamber (C) to receive at least one garment (G),
- a heating system (HS) for heating said at least one garment (G),
- a lighting system (LS) for emitting inside said chamber (C), a radiation having wavelength in the range from 280 nm to 500 nm,
- a metal oxide photocatalyst element (MO) disposed inside said chamber (C) such that the metal oxide photocatalyst element (MO) can receive said radiation,
wherein the chamber (C) comprises receiving means (H, T) to receive said at least one garment (G) such that said at least one garment (G) remains spaced from said metal oxide photocatalyst element (MO), **characterized in that**:
- said lighting system (LS) and said metal oxide photocatalyst element (MO) are disposed adjacent to a same given wall of said chamber (C), said same given wall being taken among the set defined by bottom wall, lateral wall, rear wall, front wall, and top wall, and
- said metal oxide photocatalyst element (MO) takes the form of a radiation reflector (LR) disposed between said lighting system (LS) and said same given wall.

14. A garment care device (D) comprising:
- a chamber (C) to receive at least one garment (G),
- a heating system (HS) for heating said at least one garment (G),
- a lighting system (LS) for emitting inside said chamber (C), a radiation having wavelength in the range from 280 nm to 500 nm,
- a metal oxide photocatalyst element (MO) disposed inside said chamber (C) such that the metal oxide photocatalyst element (MO) can receive said radiation, wherein the chamber (C) comprises receiving means (H, T) to receive said at least one garment (G) such that said at least one garment (G) remains spaced from said metal oxide photocatalyst element (MO), **characterized in that**:
- said lighting system (LS) and said metal oxide photocatalyst element (MO) are disposed adjacent to a same given wall of said chamber (C), said same given wall being taken among the set defined by bottom wall, lateral wall, rear wall, front wall, and top wall, and
- said metal oxide photocatalyst element (MO) takes the form of a perforated cover (PC), said lighting system (LS) being disposed between said metal oxide photocatalyst element (MO) and said same given wall.

## Patentansprüche

1. Kleiderpflegevorrichtung (D), umfassend:
- eine Kammer (C) zum Aufnehmen mindestens eines Kleidungsstücks (G),
- ein Heizsystem (HS) zum Erhitzen des mindestens einen Kleidungsstücks (G),
- ein Beleuchtungssystem (LS) zum Ausstrahlen einer Strahlung mit einer Wellenlänge im Bereich von 280 nm bis 500 nm im Inneren der Kammer (C),
- ein Metalloxidfotokatalysatorelement (MO), das im Inneren der Kammer (C) angeordnet ist, so dass das Metalloxidfotokatalysatorelement (MO) die Strahlung empfangen kann,
wobei die Kammer (C) Aufnahmemittel (H, T) zum Aufnehmen des mindestens einen Kleidungsstücks (G) umfasst, so dass das mindestens eine Kleidungsstück (G) vom Metalloxidfotokatalysatorelement (MO) beabstandet bleibt, **dadurch gekennzeichnet, dass** das Metalloxidfotokatalysatorelement (MO) die Form mindestens einer Schicht aufweist, die mit einer Wand der Kammer (C) in Kontakt ist, wobei die Wand aus dem Satz genommen wird, der durch Bodenwand, Seitenwand, Rückwand, Vorderwand und obere Wand definiert ist.

2. Kleiderpflegevorrichtung (D) nach Anspruch 1, wobei das Beleuchtungssystem (LS) neben einer bestimmten Wand der Kammer (C) angeordnet ist, wobei die bestimmte Wand aus dem Satz genommen wird, der durch Bodenwand, Seitenwand, Rückwand, Vorderwand und obere Wand definiert ist.

3. Kleiderpflegevorrichtung (D) nach einem der vorstehenden Ansprüche, wobei das Metalloxidfotokatalysatorelement (MO) aus dem Satz von Metalloxiden gewählt ist, der durch Titandioxid mit der chemischen Formel TiO₂, Eisenoxid mit der chemischen Formel FeO, Eisenoxid mit der chemischen Formel Fe₂O₃ und Eisenoxid mit der chemischen Formel Fe₃O₄ definiert ist.

4. Kleiderpflegevorrichtung (D) nach einem der vorstehenden Ansprüche, weiter umfassend ein erstes Gebläse (F1) zum Zirkulieren von Luft im Inneren der Kammer (C).

5. Kleiderpflegevorrichtung (D) nach Anspruch 4, weiter umfassend eine Außenschale (ES), die die Kammer (C) umschließt, wobei das Heizsystem (HS) und das erste Gebläse (F1) in einem Luftkanal (AC) angeordnet sind, der zwischen der Außenschale (ES) und der Kammer (C), angeordnet ist, um einen Heißluftstrom in der Kammer (C) zirkulieren zu lassen.

6. Kleiderpflegevorrichtung (D) nach Anspruch 5, wobei das erste Gebläse (F1) ausgelegt ist, einen Heißluftstrom in der Kammer (C) im Bereich von 0,01 m/s bis 2 m/s zirkulieren zu lassen.

7. Kleiderpflegevorrichtung (D) nach Anspruch 4, weiter umfassend ein zweites Gebläse (F2) zum Abziehen von Luft aus der Kammer (C).

8. Kleiderpflegevorrichtung (D) nach einem der Ansprüche 1 bis 7, wobei das Beleuchtungssystem (LS) ausgelegt ist, die Strahlung mit einer Wellenlänge im Bereich von 315 nm bis 400 nm auszustrahlen.

9. Kleiderpflegevorrichtung (D) nach einem der Ansprüche 1 bis 7, wobei das Beleuchtungssystem (LS) ausgelegt ist, die Strahlung mit einer Wellenlänge im Bereich von 280 nm bis 315 nm auszustrahlen.

10. Kleiderpflegevorrichtung (D) nach einem der Ansprüche 1 bis 7, wobei das Beleuchtungssystem (LS) mehrere Beleuchtungseinheiten umfasst.

11. Kleiderpflegevorrichtung (D) nach Anspruch 8, 9 oder 10, wobei das Beleuchtungssystem (LS) ausgelegt ist, die Strahlung mit einer Beleuchtungsenergie von mindestens 2 Kilojoules/m2 auszustrahlen.

12. Verfahren zur Behandlung eines Kleidungsstücks in einer Kleiderpflegevorrichtung (D), umfassend:
- eine Kammer (C) zum Aufnehmen mindestens eines Kleidungsstücks (G),
- ein Heizsystem (HS) zum Erhitzen des mindestens einen Kleidungsstücks (G),
- ein Beleuchtungssystem (LS) zum Ausstrahlen einer Strahlung mit einer Wellenlänge im Bereich von 280 nm bis 500 nm im Inneren der Kammer (C),
- ein Metalloxidfotokatalysatorelement (MO), das im Inneren der Kammer (C) angeordnet ist, so dass das Metalloxidfotokatalysatorelement (MO) die Strahlung empfangen kann,
wobei das Verfahren den Schritt umfasst:
- Anordnen des mindestens einen Kleidungsstücks (G) in der Kammer (C), so dass das mindestens eine Kleidungsstück (G) vom Metalloxidfotokatalysatorelement (MO) beabstandet bleibt, **dadurch gekennzeichnet, dass** das Metalloxidfotokatalysatorelement (MO) die Form mindestens einer Schicht aufweist, die mit einer Wand der Kammer (C) in Kontakt ist, wobei die Wand aus dem Satz genommen wird, der durch Bodenwand, Seitenwand, Rückwand, Vorderwand und obere Wand definiert ist.

13. Kleiderpflegevorrichtung (D), umfassend:
- eine Kammer (C) zum Aufnehmen mindestens eines Kleidungsstücks (G),
- ein Heizsystem (HS) zum Erhitzen des mindestens einen Kleidungsstücks (G),
- ein Beleuchtungssystem (LS) zum Ausstrahlen einer Strahlung mit einer Wellenlänge im Bereich von 280 nm bis 500 nm im Inneren der Kammer (C),
- ein Metalloxidfotokatalysatorelement (MO), das im Inneren der Kammer (C) angeordnet ist, so dass das Metalloxidfotokatalysatorelement (MO) die Strahlung empfangen kann,
wobei die Kammer (C) Aufnahmemittel (H, T) zum Aufnehmen des mindestens einen Kleidungsstücks (G) umfasst, so dass das mindestens eine Kleidungsstück (G) vom Metalloxidfotokatalysatorelement (MO) beabstandet bleibt, **dadurch gekennzeichnet, dass**:
- das Beleuchtungssystem (LS) und das Metalloxidfotokatalysatorelement (MO) neben einer selben gegebenen Wand der Kammer (C) angeordnet sind, wobei dieselbe gegebene Wand aus dem Satz genommen wird, der durch Bodenwand, Seitenwand, Rückwand, Vorderwand und obere Wand definiert ist, und
- das Metalloxidfotokatalysatorelement (MO) die Form eines Strahlungsreflektors (LR) aufweist, der zwischen dem Beleuchtungssystem (LS) und derselben gegebenen Wand angeordnet ist.

14. Kleiderpflegevorrichtung (D), umfassend:
- eine Kammer (C) zum Aufnehmen mindestens eines Kleidungsstücks (G),
- ein Heizsystem (HS) zum Erhitzen des mindestens einen Kleidungsstücks (G),
- ein Beleuchtungssystem (LS) zum Ausstrahlen einer Strahlung mit einer Wellenlänge im Bereich von 280 nm bis 500 nm im Inneren der Kammer (C),
- ein Metalloxidfotokatalysatorelement (MO), das im Inneren der Kammer (C) angeordnet ist, so dass das Metalloxidfotokatalysatorelement (MO) die Strahlung empfangen kann, wobei die Kammer (C) Aufnahmemittel (H, T) zum Aufnehmen des mindestens einen Kleidungsstücks (G) umfasst, so dass das mindestens eine Kleidungsstück (G) vom Metalloxidfotokatalysatorelement (MO) beabstandet bleibt, **dadurch gekennzeichnet, dass**:
das Beleuchtungssystem (LS) und das Metalloxidfotokatalysatorelement (MO) neben einer selben gegebenen Wand der Kammer (C) angeordnet sind, wobei dieselbe gegebene Wand aus dem Satz genommen wird, der durch Bodenwand, Seitenwand, Rückwand, Vorderwand und obere Wand definiert ist, und
- das Metalloxidfotokatalysatorelement (MO) die Form eines perforierten Deckels (PC) aufweist, wobei das Beleuchtungssystem (LS) zwischen dem Metalloxidfotokatalysatorelement (MO) und derselben gegebenen Wand angeordnet ist.

## Revendications

1. Dispositif d'entretien de vêtement (D) comprenant :
- une chambre (C) pour recevoir au moins un vêtement (G),
- un système de chauffage (HS) pour chauffer ledit au moins un vêtement (G),
- un système d'éclairage (LS) pour émettre à l'intérieur de ladite chambre (C), un rayonnement ayant une longueur d'onde comprise dans la plage de 280 nm à 500 nm,
- un élément photocatalyseur d'oxyde métallique (MO) disposé à l'intérieur de ladite chambre (C) de sorte que l'élément photocatalyseur d'oxyde métallique (MO) peut recevoir ledit rayonnement,
- dans lequel la chambre (C) comprend des moyens de réception (H, T) pour recevoir ledit au moins un vêtement (G) de sorte que ledit au moins un vêtement (G) reste espacé dudit élément photocatalyseur d'oxyde métallique (MO), **caractérisé en ce que** ledit élément photocatalyseur d'oxyde métallique (MO) prend la forme d'au moins une couche en contact avec une paroi de ladite chambre (C), ladite paroi étant prise parmi l'ensemble défini par une paroi inférieure, une paroi latérale, une paroi arrière, une paroi avant, et une paroi supérieure.

2. Dispositif d'entretien de vêtement (D) selon la revendication 1, dans lequel ledit système d'éclairage (LS) est disposé de manière adjacente à une paroi donnée de ladite chambre (C), ladite paroi donnée étant prise parmi l'ensemble défini par une paroi inférieure, une paroi latérale, une paroi arrière, une paroi avant et une paroi supérieure.

3. Dispositif d'entretien de vêtement (D) selon l'une quelconque des revendications précédentes, dans lequel ledit élément photocatalyseur d'oxyde métallique (MO) est choisi parmi l'ensemble d'oxydes métalliques définis par du dioxyde de titane ayant une formule chimique TiO₂, de l'oxyde de fer ayant une formule chimique FeO, de l'oxyde de fer ayant une formule chimique Fe₂O₃, et de l'oxyde de fer ayant une formule chimique Fe₃O₄.

4. Dispositif d'entretien de vêtement (D) selon l'une quelconque des revendications précédentes, comprenant en outre un premier ventilateur (F1) pour faire circuler de l'air à l'intérieur de ladite chambre (C).

5. Dispositif d'entretien de vêtement (D) selon la revendication 4, comprenant en outre une coque externe (ES) comprenant ladite chambre (C), ledit système de chauffage (HS) et ledit premier ventilateur (F1) étant disposé dans un canal d'air (AC) formé entre ladite coque externe (ES) et ladite chambre (C), pour faire circuler un flux d'air chaud dans ladite chambre (C).

6. Dispositif d'entretien de vêtement (D) selon la revendication 5, dans lequel ledit premier ventilateur (F1) est conçu pour faire circuler un flux d'air chaud dans ladite chambre (C) dans la plage de 0,01 m/s à 2 m/s.

7. Dispositif d'entretien de vêtement (D) selon la revendication 4, comprenant en outre un second ventilateur (F2) pour attirer de l'air hors de ladite chambre (C).

8. Dispositif d'entretien de vêtement (D) selon l'une quelconque des revendications 1 à 7, dans lequel ledit système d'éclairage (LS) est conçu pour émettre ledit rayonnement avec une longueur d'onde dans la plage de 315 nm à 400 nm.

9. Dispositif d'entretien de vêtement (D) selon l'une quelconque des revendications 1 à 7, dans lequel ledit système d'éclairage (LS) est conçu pour émettre ledit rayonnement avec une longueur d'onde dans la plage de 280 nm à 315 nm.

10. Dispositif d'entretien de vêtement (D) selon l'une quelconque des revendications 1 à 7, dans lequel ledit système d'éclairage (LS) comprend une pluralité d'unités d'éclairage.

11. Dispositif d'entretien de vêtement (D) selon l'une quelconque des revendications 8, 9 ou 10, dans lequel ledit système d'éclairage (LS) est conçu pour émettre ledit rayonnement avec une énergie d'éclairage d'au moins 2 kilo Joules/m².

12. Procédé de traitement d'un vêtement dans un dispositif d'entretien de vêtement (D) comprenant :
- une chambre (C) pour recevoir au moins un vêtement (G),
- un système de chauffage (HS) pour chauffer ledit au moins un vêtement (G),
- un système d'éclairage (LS) pour émettre à l'intérieur de ladite chambre (C), un rayonnement ayant une longueur d'onde dans la plage de 280 nm à 500 nm,
- un élément photocatalyseur d'oxyde métallique (MO) disposé à l'intérieur de ladite chambre (C) de sorte que l'élément photocatalyseur d'oxyde métallique (MO) puisse recevoir ledit rayonnement,
le procédé comprenant l'étape consistant à :
- disposer ledit au moins un vêtement (G) dans la chambre (C) de sorte que ledit au moins un vêtement (G) reste espacé de l'élément photocatalyseur d'oxyde métallique (MO), **caractérisé en ce que** ledit élément photocatalyseur d'oxyde métallique (MO) prend la forme d'au moins une couche en contact avec une paroi de ladite chambre (C), ladite paroi étant prise parmi l'ensemble défini par la paroi inférieure, la paroi latérale, la paroi arrière, la paroi avant et la paroi supérieure.

13. Dispositif d'entretien de vêtement (D) comprenant :
- une chambre (C) pour recevoir au moins un vêtement (G) ;
- un système de chauffage (HS) pour chauffer ledit au moins un vêtement (G),
- un système d'éclairage (LS) pour émettre à l'intérieur de ladite chambre (C), un rayonnement ayant une longueur d'onde dans la plage de 280 nm à 500 nm,
- un élément photocatalyseur d'oxyde métallique (MO) disposé à l'intérieur de ladite chambre (C) de sorte que l'élément photocatalyseur d'oxyde métallique (MO) puisse recevoir ledit rayonnement,
dans lequel la chambre (C) comprend des moyens de réception (H, T) pour recevoir ledit au moins un vêtement (G), de sorte que ledit au moins un vêtement (G) reste espacé dudit élément photocatalyseur d'oxyde métallique (MO), **caractérisé en ce que** :
- ledit système d'éclairage (LS) et ledit élément photocatalyseur d'oxyde métallique (MO) sont disposés de manière adjacente à une même paroi donnée de ladite chambre (C), ladite même paroi donnée étant prise parmi l'ensemble défini par une paroi inférieure, une paroi latérale, une paroi arrière, une paroi avant, et une paroi supérieure, et
- ledit élément photocatalyseur d'oxyde métallique (MO) prend la forme d'un réflecteur de radiation (LR) disposé entre ledit système d'éclairage (LS) et ladite même paroi donnée.

14. Dispositif d'entretien de vêtement (D) comprenant :
- une chambre (C) pour recevoir au moins un vêtement (G),
- un système de chauffage (HS) pour chauffer ledit au moins un vêtement (G),
- un système d'éclairage (LS) pour émettre à l'intérieur de ladite chambre (C), un rayonnement ayant une longueur d'onde dans la plage de 280 nm à 500 nm,
- un élément photocatalyseur d'oxyde métallique (MO) disposé à l'intérieur de ladite chambre (C) de sorte que l'élément photocatalyseur d'oxyde métallique (MO) puisse recevoir ledit rayonnement, dans lequel la chambre (C) comprend des moyens de réception (H ; T) pour recevoir ledit au moins un vêtement (G) de sorte que ledit au moins un vêtement (G) reste espacé dudit élément photocatalyseur d'oxyde métallique (MO), **caractérisé en ce que**
- ledit système d'éclairage (LS) et ledit élément photocatalyseur d'oxyde métallique (MO) sont disposés de manière adjacente à une même paroi donnée de ladite chambre (C), ladite même paroi donnée étant prise parmi l'ensemble défini par une paroi inférieure, une paroi latérale, une paroi arrière, une paroi avant, et une paroi supérieure, et
- ledit élément photocatalyseur d'oxyde métallique (MO) prend la forme d'un couvercle perforé (PC), ledit système d'éclairage (LS) étant disposé entre ledit élément photocatalyseur d'oxyde métallique (MO) et ladite même paroi donnée.
